# EUROPEAN PATENT APPLICATION

(11) **EP 1 512 369 A1**
(43) Date of publication of application: **09.03.2005**
(21) Application number: 03733314.3
(22) Date of filing: 06.06.2003
(51) Int. Cl.: A61B 5/00, A61B 5/22, G06F 17/60, H04M 1/00

(54) **PORTABLE TERMINAL DEVICE AND LIFE STYLE-RELATED DISEASE PATIENT-MEDICAL INSTITUTION COOPERATION SYSTEM**

(30) Priority: 07.06.2002 JP 2002167137
(71) Applicant: PRECISION SYSTEM SCIENCE CO., LTD., Matsudo-shi, Chiba, 271-0064 (JP)
(72) Inventor: TAJIMA, Hideji, Precision System Science Co., Ltd., Matsudo-shi, Chiba 271-0064 (JP)
(74) Representative: Bohnenberger, Johannes, Dr.
(86) International application number: PCT/JP2003/007227
(87) International publication number: WO 2003/103485

(57) **Abstract**

There is provided a portable terminal device, and a lifestyle-related disease patient - medical institution cooperation system, which collects a user's biological data and life data, so that it is possible to quickly receive advice from a specialist, and data accumulation and analysis over a long period is possible, to accurately comprehend deviations in lifestyle habits and the life of a patient, and to give a highly reliable medical service.

The invention is a terminal device which can be carried by the user, comprising: a measuring unit which measures the user's in-vivo substance so as to obtain biological data; an input unit which can input life data including at least the user's food intake contents; a data storage unit which readably stores basic data including the biological data and the life data, and a display unit which displays the data.

## Description

### TECHNICAL FIELD

The present invention relates to a portable terminal device and a lifestyle-related disease patient - medical institution cooperation system, specifically to a lifestyle-related disease patient - medical institution cooperation system wherein portable terminal devices carried and used by patients, and a portable terminal device group, an information processor in a patient data management center which manages the data sent by patients, and a terminal device group of medical institutes which can use the patient data in the patient data management center to perform medical services with respect to the patients, are connected via the Internet.

### BACKGROUND ART

Recently, a number of patients suffering from lifestyle-related diseases such as diabetes caused by unhealthy lifestyle habits such as overeating, overdrinking, irregular meals, unbalanced meal contents, and insufficient exercise, has been dramatically expanding. According to statistics, some reports say that almost half of the Japanese population are susceptible to diabetes. Since this lifestyle-related disease is caused by long term lifestyle habits including dietary habits, it is necessary to carefully record a patient's life including everyday dietary habits over a long period, and to examine the contents, in order to prevent the lifestyle-related disease or to treat it. Moreover, it is necessary to show the record to specialists such as doctors to receive their advice.

However, the reality is that everyday work and odd jobs interfere with recording the life including everyday dietary habits. Moreover, in some cases it may be necessary to examine the contents of the life including the food contents at each time. However it is not possible to consult with specialists such as doctors or to get constant monitoring, at each meal time or in close relationship with lifestyle habits.

Therefore, the present invention has been made in order to solve the above problems, with a first object of providing a portable terminal device and a lifestyle-related disease patient - medical institution cooperation system which collects data on a patient's life such as food intake contents of a patient (or user), or data on an in-vivo substance, to thereby enable a quick understanding of the patient's living conditions and the lifestyle habits so as to improve this, or to receive advice from a specialist.

A second object is to provide a portable terminal device and a lifestyle-related disease patient - medical institution cooperation system which enables long term data accumulation and analysis, to accurately comprehend deviations or trends in lifestyle habits, dietary habits, and the like of a patient (or user), so as to improve the lifestyle habits and to give a highly reliable medical service.

A third object is to provide a portable terminal device and a lifestyle-related disease patient - medical institution cooperation system which collects a patient's (or user's) data including food intake contents closely related to the patient, almost continuously in real time while the patient is living a daily life, so as to obtain data which faithfully tracks the patient's daily lifestyle habits.

A fourth object is to provide a portable terminal device and a lifestyle-related disease patient - medical institution cooperation system which obtains in combination the contents of a patient's (or user's) life data such as food intake contents, and biological data on in-vivo substances, so as to further process this and obtain clinical data which is easy to use, thus making it possible to receive highly reliable medical service.

A fifth object is to provide a portable terminal device and a lifestyle-related disease patient - medical institution cooperation system which makes it possible to receive a medical service as if a specialist such as doctor is constantly caring for the patient.

### DISCLOSURE OF THE INVENTION

In order to solve the above technical problems, a first aspect of the present invention is a portable terminal device which can be carried by a user, comprising: an input unit which can input life data including at least the user's food intake contents; a data storage unit which readably stores basic data including the life data; and a display unit which displays the data.

Here, 'user' is not necessarily a patient suffering from a lifestyle-related disease and also includes a person who wants to prevent a lifestyle-related disease.

'Food intake contents' is mainly data comprising the food type and the quantity. The reason why the life data includes the food intake contents is that food ingested substantially periodically into the human body greatly affects lifestyle-related diseases.

The aforementioned 'life data' is the data related to a specified predetermined lifestyle-related disease, and includes in addition to the food intake contents, data related to human metabolism for the user's life, for example data on the user's exercise quantity, user's sleep time, user's work or activity time, user's intake of luxury goods such as tobacco, and tooth brushing time, frequency and method.

'Input unit' includes for example, various keys, touch panels, and the like. In order to input the life data, there are cases where it is directly input, where it is input by selecting the item displayed on the screen of the display unit, where the food type and the quantity are input as they are, and where it is converted into the data of the relevant calorie amount and then input. In the case where it is input by selecting the item displayed on the screen of the display unit, the user readily input the data and the data is readily analyzed. A part of the life data specified by the medical institute or according to the user's desire may be automatically input into a certain measuring apparatus that measures the life data, for example a counter or the like.

Storage into the 'data storage unit' includes a case of temporary storage for sending or inputting, and a case of long term storage. It also includes comments showing the user's symptoms and conditions, and data selected and shown from displayed choices.

Moreover, the 'display unit' may have a screen of liquid crystal or the like, which automatically shows the measurement result and/or the date and time of the measurement and/or the input result on the display unit.

Furthermore, a control unit is provided in the portable terminal device. The control unit comprises a CPU, various memories, or timer apparatus, and the like. For example, various data input by the user or input previously is stored into the memories. Predetermined programs which perform various controls such as input processing of various data, data display, data conversion, control of data transfer, data accumulation, analysis of pertinent data, creating clinical data based on the pertinent data, or creating source material by statistical processing, are also stored therein. Data conversion includes for example, conversion of the input data into calorific value on a daily basis, and conversion of the exercise quantity into calorific value so as to calculate the calorific value per day and to calculate the quantity of various constituents in the ingested food.

According to the first aspect of the present invention, it is possible to input the life data including the user's food intake contents, so that the basic data required for analyzing the user's lifestyle habits can be obtained certainly with high reliability.

According to the present invention, since only inputting of the life data is performed, a device with a simple structure, low price and high portability can be provided.

Moreover, by carrying it at all times, highly reliable data closely related to the life can be obtained. Furthermore, when inputting the data, it is possible to use the display unit in order to display the items to be selected on the display unit of the portable terminal device, to give an explanation to facilitate the input of the life data, and to give detailed instructions and information to the user.

A second aspect of the present invention is a portable terminal device wherein the aforementioned portable terminal device has a measuring unit which can measure a user's in-vivo substance to obtain biological data, and the basic data includes the biological data and the life data.

Here, 'in-vivo substance' means a substance which is generated and stored in vivo, or exists in vivo, and a substance which relates to a predetermined lifestyle-related disease specified by a medical institute or according to the user's desire. It includes for example, sugar, fat, active oxygen, various minerals such as sodium, magnesium, iron, zinc, copper, potassium, calcium, aluminum, chlorine, amino acids, proteins, vitamins, red blood corpuscles, white blood corpuscles, lactic acids, alcohol, hormones, virus, bacterium, immunity substances, DNA, cells, dental plaques, dental caries bacterium, and periodontal bacterium, in the blood, urine, saliva, or other body fluids, or in the mouth, and any combination thereof. The in-vivo substance to be measured is determined by the user's symptoms, the user's desire, or the doctor's instructions.

'Biological data' is data related to the contents, such as the existence or nonexistence of the in-vivo substance of the user, the amount, comparison with a standard value, and discrimination of normal or abnormal. It is also possible for example that the predetermined biological data specified by the medical institute or according to the user's desire is measured by the measuring apparatus, and other predetermined biological data is input from the input unit.

The 'measuring unit' may be incorporated into the portable terminal device, or may be a unit which is detachably connected to the portable terminal device, and is capable of transferring information from the outside.

According to the second aspect of the present invention, the biological data on the in-vivo substance and the basic data associated with the life data including food intake contents can be collected in close relationship to the user. Therefore, the user's lifestyle habits and the influence thereof can be reliably obtained, and made use of for the prevention and the treatment of lifestyle-related diseases.

Furthermore, by collecting the life data including food intake contents closely related to the patient, almost continuously in real time while the patient is living a daily life, data which faithfully tracks the patient's daily lifestyle habits can be obtained.

Moreover, by storing the basic data into the portable terminal device, the basic data can be shown to the medical institute collectively for each fixed period, and can be used by the patient him/herself. By storing and accumulating the basic data, deviations or trends in lifestyle habits can be accurately determined.

Furthermore, it is possible to receive a highly effective medical service as if a doctor is constantly caring for the patient.

By automatic display of the measurement results, the measurement time and date, and the like on the display unit, the user quickly knows the measurement results so as to make use of them for quick improvement of his/her lifestyle habits.

A third aspect of the present invention is a portable terminal device wherein the data storage unit is removably provided with respect to the portable terminal device.

Here, 'removable data storage unit' includes for example, a semiconductor memory, a flexible disk, a CD, and the like. The removed data storage unit is directly passed to the doctor to be analyzed, for example periodically or when the user goes the medical institute.

According to the third aspect of the present invention, the data storage unit is provided removably from the portable terminal device so that the data storage unit can be left at the medical institute when the user goes the medical institute such as a hospital, or visits the specialist, to ask for a detailed analysis.

A fourth aspect of the present invention is a portable terminal device wherein a data transfer unit which transfers data between the outside and the portable terminal device is provided in the portable terminal device.

Here, 'data transfer unit' includes for example, a communication unit which transmits and receives data, or a connection unit which includes a terminal for connecting between a portable telephone and the main body unit so as to enable information transmission therebetween. The destination of the data is for example an information processor. The information processor processes based on the sent basic data, calculates other data, accumulates it and creates clinical data described later. The results obtained in such a manner are for example, further sent to the medical institute, or returned to the portable terminal device.

According to the fourth aspect of the present invention, by providing in the portable terminal device, the data transfer unit which reads out the data stored in the data storage unit and transfers it to outside of the portable terminal device, the data can be stored and accumulated in a personal-computer main body in the user's home, or analyzed in detail by the main body unit. Moreover, by showing it to a medical institute such as a management center, a hospital or the like, or to a specialist, it is possible to request management of the data, or a detailed analysis. Furthermore, by providing a communication unit as the data transfer unit, the data can be sent to the medical institute or the specialist in real time to request analysis and diagnosis, so that it is possible to quickly receive advice from the medical institute or the specialist.

A fifth aspect of the present invention is a portable terminal device wherein the biological data includes measurement time point data showing a measurement time point when the in-vivo substance is measured by the measuring unit.

Here, 'measurement time point data' includes for example the date and the time of the measurement. The data may be automatically measured by incorporating a clock into the portable terminal device.

According to the fifth aspect of the present invention, by including the measurement time point for when the in-vivo substance is measured, correlation with the time can be seen, so that accurate and highly useful basic data on the patient can be obtained.

In a sixth aspect of the present invention the measuring unit in the portable terminal device is an in-vivo substance measuring apparatus which is detachably and data transferably connected to the portable terminal device main body.

Here, 'in-vivo substance measuring apparatus' includes for example, a blood glucose level measuring apparatus, a protein quantity measuring apparatus, an active oxygen measuring apparatus, a mineral composition measuring apparatus, and the like, being a portable device.

According to the sixth aspect of the present invention, by providing the in-vivo substance measuring apparatus detachably connected to the portable terminal device, the in-vivo substance measuring apparatus can be removed from the portable terminal device when it is not necessary, so that the portable terminal device can be made compact to increase portability. Moreover, since various in-vivo substance measuring apparatuses can be selected to use according to purpose, generality is high.
A seventh aspect of the present invention is a portable terminal device wherein the display unit and the input unit or data transfer unit in the portable terminal device use a display unit and an input unit or a communication unit of a portable telephone which is detachably and data transferably connected to the portable terminal device main body.

According to the seventh aspect of the present invention, by using the portable telephone for the display unit and the input unit or the data transfer unit in the portable terminal device, the structure of the portable terminal device can be simplified, further increasing portability and enabling it to be provided at a low price.

An eighth aspect of the present invention is a portable terminal device wherein the input unit which inputs the food intake contents, previously sets a food intake pattern according to special conditions and a pattern based on lifestyle habits of breakfast, lunch, and dinner, so as to suit the user's dietary habits, and classifies fixed units for each food into levels such as large quantity, medium quantity, and small quantity, to enable selection thereof.

In this case, in order to determine the user's lifestyle habits or to create clinical data, a conversion table data which associates between the food intake pattern and the corresponding calorific value is previously provided in the memory of the portable terminal device. Furthermore, it is set for respective users, or general standard calorific data for each fixed period such as each day is provided in the memory. Moreover, an arithmetic program which calculates the calorific value and creates the clinical data based on the conversion table data or the standard calorific data, is provided in the memory. Furthermore, food composition table data is previously set in the memory. Also there is provided an arithmetic program which calculates the quantity of the various constituents in the ingested food, for example minerals such as salt, calcium, and the like, dietary fiber, and various vitamins, based on this food composition table data, so as to create the clinical data. Moreover, it is set for respective users, or general standard composition data for the various constituents for each fixed period such as each day is provided in the memory. The food composition table is a table which shows the various constituents such as the proteins, saccharides, dietary fiber, various minerals, various vitamins contained in the various foods, the presence or amount of substances, and the calorific value. An example includes the Standard Tables of Food Composition in Japan.

When inputting the data, this is preferably input with the pattern displayed on the display unit. Moreover, the input results or the calculation results are displayed on the display unit.

According to the eighth aspect of the present invention, when inputting the food intake contents, they are input for each fixed pattern. Therefore, they are readily input and analyzed. Particularly, if the pattern is displayed on the display unit or the like, the food intake contents can be even more readily input.

A ninth aspect of the present invention is a portable terminal device wherein the basic data includes exercise data showing a user's exercise quantity.

According to the ninth aspect of the present invention, by including the exercise data showing the exercise quantity of the patient, in the basic data, more highly reliable basic data can be obtained.

A tenth aspect of the present invention is a portable terminal device comprising an exercise quantity measuring apparatus which is formed integrally with the portable terminal device or separately from the portable terminal device, and measures the exercise quantity of a user carrying the device so as to obtain a part or all of the exercise data.

Here, the 'exercise quantity measuring apparatus' is a pedometer which measures for example the number of steps so as to calculate the exercise quantity such as the calorific amount by the number of steps itself, or from an appropriate conversion equation. 'Integrally or separately' includes the case where the exercise quantity measuring apparatus is provided detachably and data transferably connected to the portable terminal device.

According to the tenth aspect of the present invention, by using the exercise quantity measuring apparatus formed integrally with or separately from the portable terminal device, even more reliable exercise data can be obtained.

An eleventh aspect of the present invention is a portable terminal device wherein a part or all of the specified exercise data is input from the input unit.

Here, 'exercise data' includes the data itself measured by the exercise quantity measuring apparatus, or for example the number of holes, the number of swings, or the number of balls hit in golf, the time spend for sports such as tennis, the degree of severity of the sports, the number of repeated exercises such as push-ups, the time or the distance for running or walking, or may be the data thereof converted into heat quantity values such as calorific values. Regarding the conversion, for example an exercise quantity conversion table data showing the relation between the contents of an exercise and the calorific value is previously provided in the memory, and conversion is made based on this exercise quantity conversion table. Otherwise, the data is converted based on a predetermined arithmetic equation by inputting variables constituting the arithmetic equation.

According to the eleventh aspect of the present invention, by inputting the exercise data from the input unit, various exercise quantities, for example the number of holes, the number of swings, or the number of balls hit in golf, the time spend for sports such as tennis, the time and the distance for running, or the time for walking, can be input, so that the various exercise quantities matching the user's lifestyle habits can be measured.

A twelfth aspect of the present invention is a portable terminal device wherein the portable terminal device comprises a clinical data creating section which creates clinical data based on the basic data.

The 'clinical data created based on the basic data' is data created for facilitating use in a medical institute such as by a doctor, or by the user, for example the results for the quantity of ingested constituents, calculated by timewise correlation of the biological data and the life data, or based on the conversion table data or the standard data.

According to the twelfth aspect of the present invention, for example by combining the measured value related to the user's in-vivo substance, and the contents of the life data including food intake, clinical data which is more easy to use can be created, so that a highly reliable medical service can be received.

A thirteenth aspect of the present invention is a portable terminal device wherein the clinical data creating section creates a table showing a causal relation with food intake, and graphs the data corresponding to clinical analysis.

According to the thirteenth aspect of the present invention, by creating the table showing a causal relation with food intake and graphing, based on the basic data, the data is made easy to use by the user and the medical institute.

A fourteenth aspect of the present invention is a portable terminal device wherein the clinical data creating section creates an acceptable intake on the day, based on a difference between a limited calorific intake per day and ingested calories on the day.

According to the fourteenth aspect of the present invention, by quickly creating the acceptable intake on the day based on the difference between the limited calorific intake per day and ingested calories on the day, and sending it to the patient's portable terminal device, the patient can be quickly oriented in a direction to improve his/her lifestyle habits.

A fifteenth aspect of the present invention is a portable terminal device wherein the portable terminal device comprises a source material creating section which creates source material having the accumulated basic data or clinical data statistically processed. Here, 'statistical processing' includes for example, yearly or monthly aggregation of the type and the quantity of ingested food, aggregation of the exercise quantity, or calculation of the mean intake, so as to create source material such as tables or graphs.

According to the fifteenth aspect of the present invention, by statistically processing basic data or clinical data accumulated over a long period, trends or deviations in a user's lifestyle habits can be accurately comprehended so as to contribute to fundamental changes in the lifestyle habits.

A sixteenth aspect of the present invention is a lifestyle-related disease patient - medical institution cooperation system having; a portable terminal device group comprising portable terminal devices which can be carried by users, an information processor in a patient data management center which manages data sent by the portable terminal device group, and a medical treatment terminal device group in medical institutions which can use the data in the patient data management center and perform medical services with respect to the user, connected via an network. It is a lifestyle-related disease patient - medical institution cooperation system wherein the portable terminal device comprises: an input unit which can input life data including at least the user's food intake contents; a display unit which displays the data; and a communication unit which sends basic data including the life data and receives medical data, the information processor comprises: a clinical data creating section which creates clinical data based on the basic data; a clinical database which accumulates the clinical data; and a communication unit which receives the basic data and sends the clinical data, and the medical treatment terminal device comprises a communication unit which receives the clinical data and sends medical data created based on the clinical data.

Here, 'network' includes various networks wherein a communication network is formed by wireless communication means such as the Internet, an intranet, and the like. Moreover, the description of terms which have been already explained such as life data, biological data, and basic data is omitted hereunder.

'Medical institute' may be a family doctor of the patient, an institute where the patient goes, or for example a medical institute which is previously selected by the patient. The medical institute is not necessarily limited to one medical institute such as a hospital, a physician's office, a clinic, a dental clinic, an infirmary, a health center, and the like, and may be a plurality of medical institutes, or may be a related institute or individual, that performs semi-medical care. Moreover, the related institute or individual may include not only a doctor or a dentist, but also other specialists such as a national registered dietitian.

'Medical service' includes treatment, manipulation, diagnosis, prevention, or various advice to the patient.

Moreover, a clinical data search unit which searches for clinical data in a clinical database so that the medical treatment terminal device receives the search results, may be provided in the medical treatment terminal device. Furthermore, the medical data created based on the clinical data is sent to the portable terminal device directly or via the information processor in the patient data center. Otherwise, the medical data may be sent to the information processor simultaneously with the portable terminal device.
Accordingly, the patient data management center stores and manages the data and uses this for creating the source material, so that the medical service can be made easy to use by the patient.

Normally, the portable terminal device sends the basic data to the information processor and receives the medical data from the medical treatment terminal device or the information processor. Moreover, the information processor receives the basic data from the portable terminal device and sends the clinical data to the medical treatment terminal device.

When sending to the 'medical treatment terminal device group', for example the data is distributed and sent to; a medical treatment terminal device which is previously specified by the user or the information processor, a medical treatment terminal device which the information processor determines by considering the specialty according to the data contents, or which is specified by the user, or to a relevant medical treatment terminal device according to instructions from a medical institute.

According to the sixteenth aspect of the present invention, by connecting the portable terminal device to the patient data management center and the medical institute via the network, it is possible to provide a system wherein the user can send the basic data and receive the advice from a specialist quickly or in real time

Moreover, it enables accumulation and analysis of data over a long period, and accurately comprehends deviations or trends in lifestyle habits and dietary habits of the patient, so as to give a highly reliable medical service.

Furthermore, by collecting the life data including food intake contents closely related to the patient, almost continuously in real time while living a daily life, it becomes possible to obtain data which faithfully tracks the patient's daily dietary habits.

Furthermore, it is possible to receive a highly effective medical service as if a doctor is constantly caring for the patient.

A seventeenth aspect ofthe present invention is a lifestyle-related disease patient - medical institution cooperation system wherein the portable terminal device has a measuring unit which can measure a user's in-vivo substance to obtain biological data, and the basic data includes the biological data and the life data.

According to the seventeenth aspect of the present invention, the biological data on the in-vivo substance and the basic data associated with the life data including food intake contents can be collected in close relationship to the user. Therefore, the user's lifestyle habits and the influence thereof can be reliably obtained, and made use of for the prevention and the treatment of lifestyle-related diseases.

Furthermore, by collecting the life data including food intake contents closely related to the patient, almost continuously in real time while the patient is living a daily life, data which faithfully tracks the patient's daily lifestyle habits can be obtained.

Moreover, by storing the basic data into the portable terminal device, the basic data can be shown to the medical institute collectively for each fixed period, and can be used by the patient him/herself. By storing and accumulating the basic data, deviations or trends in lifestyle habits can be accurately determined.

Furthermore, it is possible to receive a highly effective medical service as if a doctor is constantly caring for the patient.

An eighteenth aspect of the present invention is a lifestyle-related disease patient - medical institution cooperation system wherein the data storage unit is removably provided with respect to the portable terminal device.

According to the eighteenth aspect of the present invention, the data storage unit is provided removably from the portable terminal device so that the data storage unit can be left at the medical institute when the user goes the medical institute such as a hospital, or visits the specialist, to ask for a detailed analysis.

A nineteenth aspect of the present invention is a lifestyle-related disease patient - medical institution cooperation system wherein the biological data includes measurement time point data for when the in-vivo substance is measured by the measuring unit.

According to the nineteenth aspect of the present invention, by including the measurement time point for when the in-vivo substance is measured, correlation with the time can be seen so that accurate and highly useful basic data on the patient can be obtained.

A twentieth aspect of the present invention is a lifestyle-related disease patient - medical institution cooperation system, wherein the measuring unit in the portable terminal device is an in-vivo substance measuring apparatus which is detachably and data transferably connected to the portable terminal device main body.

According to the twentieth aspect of the present invention, by providing the in-vivo substance measuring apparatus detachably connected to the portable terminal device, the in-vivo substance measuring apparatus can be removed from the portable terminal device when it is not necessary, so that the portable terminal device can be made compact to increase portability. Moreover, since various in-vivo substance measuring apparatuses can be selected to use according to purpose, generality is high.

A twenty first aspect of the present invention is a lifestyle-related disease patient - medical institution cooperation system wherein the display unit and the input unit or communication unit in the portable terminal device uses a display unit and an input unit or a communication unit of a portable telephone which is detachably and data transferably connected to the portable terminal device main body.

According to the twenty first aspect of the present invention, by using the portable telephone for the display unit and the input unit or the data transfer unit in the portable terminal device, the structure of the portable terminal device can be simplified, further increasing portability and enabling it to be provided at low price.

A twenty second aspect of the present invention is a lifestyle-related disease patient - medical institution cooperation system wherein the input unit which inputs the food intake contents, previously sets a food intake pattern according to special conditions and a pattern based on lifestyle habits of breakfast, lunch, and dinner, so as to suit the user's dietary habits, and classifies fixed units for each food into levels such as large quantity, medium quantity, and small quantity, to enable selection thereof. In order to analyze the input, the information processor stores the abovementioned conversion table data, the standard calorific data, the food composition table data, or standard value data in the memory.

According to the twenty second aspect of the present invention, when inputting the food intake contents, they are input for each fixed pattern. Therefore, they are readily input and analyzed. Particularly, if the pattern is displayed on the display unit or the like, the food intake contents can be even more readily input.

A twenty third aspect of the present invention is a lifestyle-related disease patient - medical institution cooperation system wherein the basic data includes exercise data showing a user's exercise quantity

According to the twenty third aspect of the present invention, by including the exercise data showing the exercise quantity of the patient in the basic data, more highly reliable basic data can be obtained.

A twenty fourth aspect of the present invention is a lifestyle-related disease patient - medical institution cooperation system comprising an exercise quantity measuring apparatus which is formed integrally with the portable terminal device or separately from the portable terminal device, and measures the exercise quantity of a user carrying the device so as to obtain a part or all of the exercise data.

According to the twenty fourth aspect of the present invention, by using the exercise quantity measuring apparatus formed integrally with or separately from the portable terminal device, even more reliable exercise data can be obtained.

A twenty fifth aspect of the present invention is a lifestyle-related disease patient - medical institution cooperation system wherein a part or all of the exercise data is input from the input unit. Moreover, the information processor has exercise quantity conversion table data or an arithmetic program in the memory.

According to the twenty fifth aspect of the present invention, by inputting the exercise data from the input unit, various exercise quantities can be input, enabling correspondence with the user's various lifestyle habits.

A twenty sixth aspect of the present invention is a lifestyle-related disease patient - medical institution cooperation system wherein the information processor sends a necessary part of the clinical data created by the clinical data creating section to the medical treatment terminal device group or the portable terminal device group.

According to the twenty sixth aspect of the present invention, the information processor sends a necessary part of the clinical data created by the clinical data creating section to the medical treatment terminal device group or the portable terminal device group. Therefore, the medical institute receives clinical data which is made more easy to use compared to the case of receiving the basic data directly from the portable terminal device, so that it is possible to receive accurate and quick medical service. Moreover, the patient can receive a medical service which can be quickly made to correspond and which is effective.

A twenty seventh aspect of the present invention is a lifestyle-related disease patient - medical institution cooperation system wherein the clinical data creating section in the information processor creates a table showing a causal relation with food intake, and graphs the data corresponding to clinical analysis.

A twenty eighth aspect of the present invention is a lifestyle-related disease patient - medical institution cooperation system wherein the clinical data creating section in the information processor creates an acceptable intake on the day as the clinical data, based on a difference between a limited calorific intake per day and ingested calories on the day, and sends it.

According to the twenty seventh and twenty eighth aspects of the present invention, since instead of the portable terminal device itself, the information processor in the patient data management center creates the table, the graph, and calculates the acceptable intake, the function of the portable terminal device itself can be simplified, so that the portable terminal device is made easy to operate and can be provided at a low price.

A twenty ninth aspect of the present invention is a lifestyle-related disease patient - medical institution cooperation system wherein in the case where a joining contract with the present system is concluded, the information processor holds the customer's personal data in the information processor.

According to the twenty ninth aspect of the present invention, in the case where the joining contract with the present system is concluded, the information processor in the patient data center holds the customer's personal data in the information processor, so as to ensure the joining with the system, and the registration and management.

### BRIEF DESCRIPTION OF THE DRAWINGS

FIG. 1 is an overall schematic diagram of a system according to an embodiment of the present invention.
FIG. 2 is a block diagram showing a portable terminal device according to the embodiment of the present invention.
FIG. 3 is a flowchart showing a measuring process according to the embodiment of the present invention.
FIG. 4 shows a screen for inputting ingested food contents according to the embodiment of the present invention.

### BEST MODE FOR CARRYING OUT THE INVENTION

A portable terminal device and a lifestyle-related disease patient - medical institution cooperation system according to an embodiment of the present invention is described based on the drawings. It should be understood that the description of the present embodiment is not to be considered as limiting to the invention unless particularly specified.

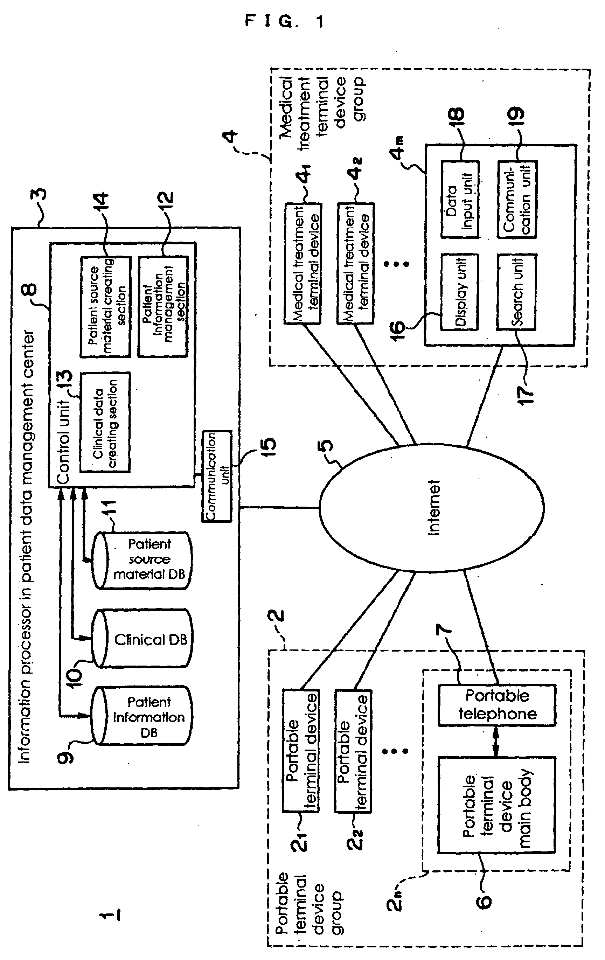

FIG. 1 is an overall schematic diagram of a lifestyle-related disease patient - medical institution cooperation system 1 according to an embodiment of the present invention.

The system 1 is a system having: a portable terminal device group 2 which is respectively carried by a plurality of previously registered patients, comprising portable terminal devices 2ₗ through 2ₙ being capable of measuring for example the blood glucose as an in-vivo substance; an information processor 3 in a patient data management center which manages the data sent by patients; and a medical treatment terminal device group 4 comprising one or more medical treatment terminal devices 4ₗ through 4ₘ of one or a plurality of medical institutions which can use the data in the information processor 3, and perform medical service with respect to the patient, all connected via the Internet 5.

The portable terminal devices 2ₗ through 2ₙ respectively have: a portable terminal device main body 6 which measures the blood glucose level being biological data, and inputs the food intake contents being life data, of a user such as a patient; and a portable telephone 7 which is electrically connected to the portable terminal device main body 6 detachably, and data transferably. The portable telephone 7 can send the basic data comprising the blood glucose level measured as the biological data, and the food intake contents input as the life data, to the information processor 3.

Moreover, the information processor 3 in the data management center manages the web site screen on the Internet 5 as a web server on the Internet 5, and shows a display to explain the outline of the lifestyle-related disease patient - medical institution cooperation system 1 on this web site screen, so as to advertise for patients at all times. The information processor 3 not only manages the web site screen, but also has: a control unit 8 in the information processor 3 which variously controls the data on the patient; a patient information database 9 which stores data of the patient information on the patient him/herself, such as name, address, gender, age or date of birth, portable telephone number, bank account number, date of birth, body weight, height, and medical history or desired medical institutes; a clinical database 10 which stores the clinical data obtained based on the basic data sent from the portable terminal devices 2ₗ through 2ₙ; and a patient source material database 11 which stores source material on the patient obtained based on the clinical data.

The control unit 8 has: a patient information management section 12 which stores and registers in the patient information database 9, the patient information sent from the patient when he/she contracts to join the present system, deletes the patient information data for which the registration is cancelled due to non-payment of membership fee, and updates the patient information data; a clinical data creating section 13 which processes the basic data sent from the portable terminal devices 2ₗ through 2ₙ to create the clinical data, and stores it in the clinical database 10, and then updates or deletes; a patient source material creating section 14 which performs statistical processing based on the clinical data to create the patient source material; and a communication unit 15 which communicates with the portable terminal device group 2 and the medical treatment terminal device group 4 via the Internet 5.

Here, clinical data means the data which is made easy to use for doctors and patients by processing the basic data, for example, a table showing the causal relation with food intake, data showing acceptable intake on the day based on the difference between the limited calorific intake per day and ingested calories on the day, and the type and the quantity of various constituents in the ingested food.

Moreover, the medical treatment terminal devices 4ₗ through 4ₘ respectively have: a search unit 17 which searches in the clinical database 10 and displays the relevant clinical data on a display unit 16; a data input unit 18 which inputs advice from specialists such as doctors based on the clinical data; and a communication unit 19 which communicates with the portable terminal device group 2 and the information processor 3 via the Internet 5.

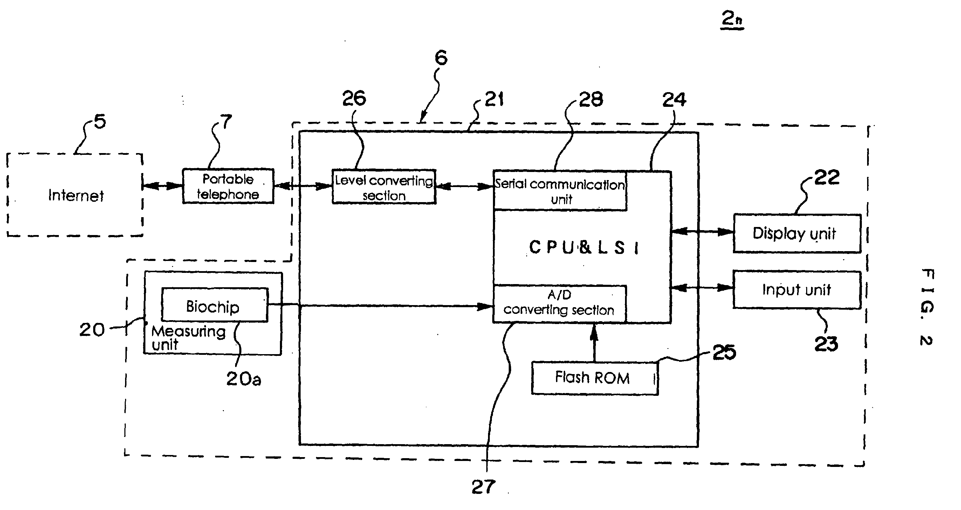

FIG. 2 is a block diagram primarily showing the portable terminal device main body 6 in the portable terminal device 2ₙ.

The portable terminal device 2ₙ comprises the portable terminal device main body 6 and the portable telephone 7. The portable terminal device main body 6 has a built-in measuring unit 20 including a biochip 20a to which is applied various reagents, which is contacted with the collected patient's blood to enable measuring of the saccharide amount in blood, that is the blood glucose level, as the in-vivo substance. Moreover, the portable terminal device main body 6 has: a control unit 21 which performs various processes such as processing data from the biochip 20a; a display unit 22 which comprises a liquid crystal for displaying the processed information and instructions from a doctor; and an input unit 23 which inputs the food intake contents, the number of steps measured by a pedometer serving as the exercise quantity measuring apparatus, and the like as the life data.

The control unit 21 has: an integrated circuit section 24 comprising a CPU and LSI; a rewritable flash ROM 25 which stores various programs and data; and a level converting section 26 serving as the data transfer unit, for connecting to the portable telephone 7. The integrated circuit section 24 has: an A/D converting section 27 which converts analog data from the biochip 20a into digital data; and a serial communication unit 28 which converts data into serial data for connecting to the portable telephone 7.

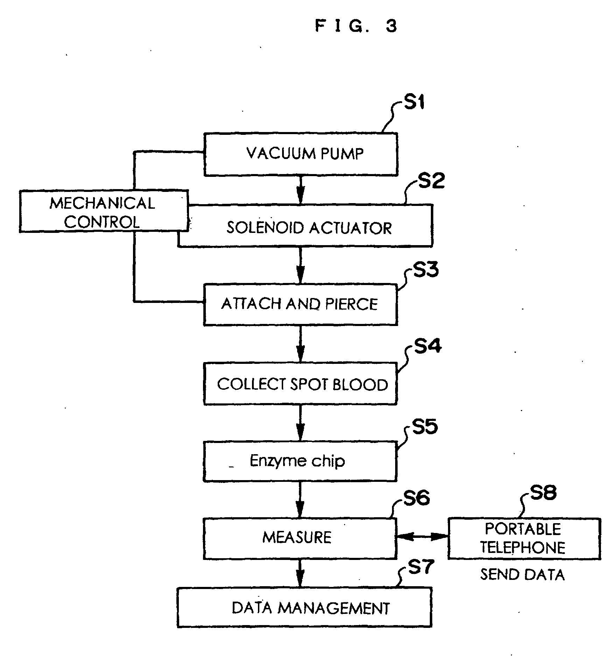

Hereunder is a description of the lifestyle-related disease patient - medical institution cooperation system 1 according to the present embodiment, based on FIG. 3 and the like.

The information processor 3 in the patient data management center of the system 1 controls the web site screen as a web server, and shows a display to explain the outline of the lifestyle-related disease patient - medical institution cooperation system 1 on this web site screen, so as to advertise for patients at all times and medical institutes at some times. If there is an application accompanied by patient information, from the patient through the patient's portable telephone 7 or other routes, in the patient data management center or in the information processor 3, and a joining contract is completed, the portable terminal device main body 6, a manual, and the like, are sent to the patient.

Moreover, the patient information is stored into the patient information database 9 by the patient information management section 12 in the control unit 8 in the information processor 3 in the patient data management center. Furthermore, the information processor 3 in the patient data management center informs the medical treatment terminal device 4ₗ of a selected predetermined medical institute, that the patient has joined and that the medical institute is in charge of the patient.

The patient who was given the portable terminal device main body 6 carries it together with the portable telephone 7 at all times. Moreover, according to instructions from the medical treatment terminal device 4ₗ or a description in the manual, he/she creates the basic data including the life data comprising the biological data of blood glucose level, the food intake contents, and the number of steps, and sends it to the information processor 3 in the patient data management center using the communication function of the portable telephone 7, by e-mail via the Internet 5.

The measuring unit 20 provided in the portable terminal device main body 6 measures the blood glucose level as the biological data. The measuring unit 20 has, for example; a vacuum pump (not shown), a solenoid actuator (not shown), and a thin needle (not shown) driven by the solenoid actuator.

As shown in FIG. 3, while in step S1 negative pressure is being applied by the vacuum pump provided in the portable terminal device main body 6, in step S2 the solenoid actuator provided in the portable terminal device main body 6 is driven. Accordingly in step S3, the thin needle provided at the tip of the actuator is slightly pierced into the human skin surface, and in step S4, a small amount of blood is drawn out and collected.

In step S5, the blood is contacted with the biochip 20a fixed with a predetermined enzyme. In step S6, the change in the biochip 20a is measured and an analog signal, being the measurement result, is converted into a digital signal by the A/D converting section 27. Then in step S7, this is stored inside the integrated circuit section 24 with the date and time of the measurement, and in step S8 is sent to the information processor 3 by the portable telephone 7 via the Internet 5 periodically after a fixed time, or immediately.

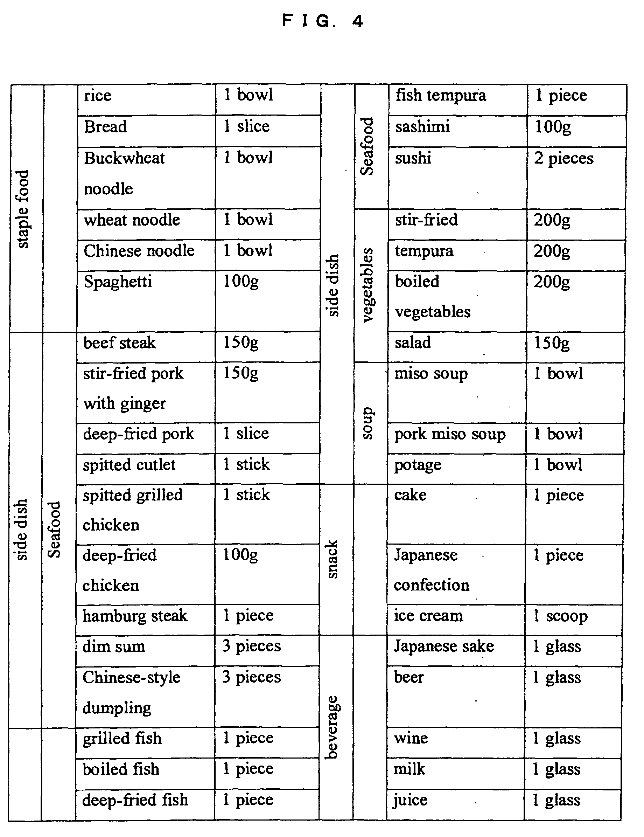

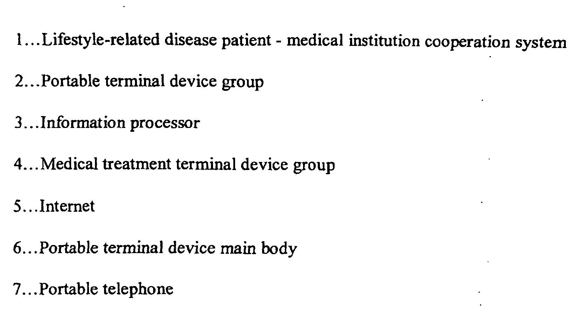

Moreover, the food contents ingested by the patient are input via the input unit 23 in the portable terminal device main body 6. When inputting the ingested food contents, for example as shown in FIG. 4, the flash ROM 25 in the portable terminal device main body 6 in the portable terminal device 2 previously has a table which associates between various foods and the calorific value with respect to the quantity, so that the ingested food and the food intake contents corresponding to the quantity can be readily input by scrolling the relevant part in the table shown in the screen, or by specifying by a cursor.

For example, a food intake pattern is previously set based on a three-meal pattern of breakfast, lunch, and dinner, and special conditions, according to the patient's dietary habits, and a table wherein the fixed unit for each displayed food is classified into about three levels such as large quantity, medium quantity, and small quantity as the ingested quantity, and an association table showing the corresponding calorific value, are stored into the flash ROM 25 in the portable terminal device main body 6 so that the ingested food quantity can be readily input by specifying on the screen. Input guidance by a voice message may be performed at the same time. An example of such a table for screen display is shown in FIG. 4.

Furthermore, the food intake pattern and the ingested calories corresponding to the quantity are stored as the association table into the portable terminal device, so that the ingested calories are automatically stored as the basic data showing the contents of the ingested food into the portable terminal device main body 6, or are immediately sent as the basic data to the information processor 3 through the portable telephone 7. Moreover, the patient inputs the consumed calories on a daily basis, obtained from the number of steps in the day which is measured by the exercise quantity measuring apparatus such as a pedometer (not shown), and then this is stored into the portable terminal device main body 6, or is sent as the basic data to the information processor 3 in the patient data management center.

The information processor which receives the basic data, creates the clinical data whereby, based on the biological data comprising the measured blood glucose level, the life data such as ingested calories and consumed calories, and the patient information, the causal relation can be understood from the food intake, the exercise quantity, and the blood glucose level associated with time by the clinical data creating section 13. At this time, it is possible to display a graph or the like. Accordingly, by combining with other information such as the recognition of a disease condition of the patient him/herself, dosage condition, and the like, it becomes the source material to determine the treatment policy of the medical institute.

Moreover, the clinical data creating section 13 in the information processor 3, creates by means of the conversion table data and the arithmetic program, the acceptable calorific intake considering the difference between the limited calorific intake per day and ingested calories, or further considering the exercise quantity, based on the basic data sent from the patient, and sends this to the portable terminal device main body 6. The quantity of various constituents ingested each day from the food is created by food composition table data and the arithmetic program. This function is not necessarily provided in the information processor 3 and it may be provided in the portable terminal device main body 6 itself.

Furthermore, the information processor 3 has a patient source material creating section 14 which creates the patient's health source material that is useful for patient's health care, or for the treatment of medical institutes, by accumulating over a comparatively long period clinical data created based on the basic data sent by the patient. Accordingly, long term patient's health care can be performed, and the dietary habits can be understood.

The medical treatment terminal device group 4 can use the clinical data search unit 17 to search for the clinical data in the clinical database 10 and display it, so as to make use of it for the treatment of the patient in the case where the patient comes to the institute. Moreover, it is possible to use the communication unit 19 so as to send the instructions and the advice from the doctor to the portable terminal device main body 6 and display them thereon.

The abovementioned respective embodiments are specifically described for better understanding of the present invention, and are not to be considered as limiting to other embodiments. Therefore, modifications can be made without departing from the spirit or scope of the present invention.

For example, in the above description, only the case was described wherein the blood glucose level was used as the biological data, and the food intake contents were used as the life data. However, this is not limited to the measurement of the blood glucose level, and the type and the combination of reagents used for the biochip may be modified, to thereby measure in-vivo substances such as proteins, amino acids, and minerals, and these can be combined and used as the biological data. Moreover, as the life data, comprehensive data including the exercise quantity data, body weight value, and others, for example, blood pressure value, pulse rate, body temperature, respiration rate, and the like may be used as the basic data, enabling correspondence with the overall lifestyle-related disease.

The basic data of the biological data or life data is not necessary sent by the portable terminal device at all times, and may be sent or brought to the medical institute regularly or irregularly. Moreover, the above components, parts, and devices, for example, the portable terminal device, the portable terminal device main body, the information processor, the medical treatment terminal device, the exercise quantity measuring apparatus, the in-vivo substance measuring apparatus, the data transfer unit, the portable telephone, the display unit, the clinical data creating section, or the like may be optionally combined while appropriately modifying these.

Furthermore, in the above description, a case was described wherein the portable terminal device has the portable telephone, and the measuring unit is incorporated into the portable terminal device. However, there may be a case where the portable terminal device has its own communication unit, and an in-vivo substance measuring apparatus which is detachably connected to the portable terminal device main body, is used as the measuring unit.

## Claims

1. A portable terminal device which can be carried by a user comprising: an input unit which can input life data including a user's food intake contents; a data storage unit which readably stores basic data including said life data; and a display unit which displays the data.

2. A portable terminal device according to claim 1, wherein said portable terminal device has a measuring unit which can measure a user's in-vivo substance to obtain biological data, and said basic data includes said biological data and said life data.

3. A portable terminal device according to either one of claim 1 and claim 2, wherein said data storage unit is removably provided with respect to said portable terminal device.

4. A portable terminal device according to any one of claim 1 through claim 3, wherein a data transfer unit which transfers data between the outside and said portable terminal device is provided in said portable terminal device.

5. A portable terminal device according to any one of claim 2 through claim 4, wherein said biological data includes measurement time point data showing a measurement time point when said in-vivo substance is measured by said measuring unit.

6. A portable terminal device according to any one of claim 2 through claim 5, wherein said measuring unit in said portable terminal device is an in-vivo substance measuring apparatus which is detachably and data transferably connected to said portable terminal device main body.

7. A portable terminal device according to any one of claim 1 through claim 6, wherein said display unit and said input unit or data transfer unit in said portable terminal device use a display unit and an input unit or a communication unit of a portable telephone which is detachably and data transferably connected to said portable terminal device main body.

8. A portable terminal device according to any one of claim 1 through claim 7, wherein said input unit which inputs said food intake contents, previously sets a food intake pattern according to special conditions and a pattern based on lifestyle habits of breakfast, lunch, and dinner, so as to suit the user's dietary habits, and classifies fixed units for each food into levels such as large quantity, medium quantity, and small quantity, to enable selection thereof.

9. A portable terminal device according to any one of claim 1 through claim 8, wherein said basic data includes exercise data showing a user's exercise quantity.

10. A portable terminal device according to any one of claim 1 through claim 9, comprising an exercise quantity measuring apparatus which is formed integrally with said portable terminal device or separately from said portable terminal device, and measures said exercise quantity of a user carrying said device so as to obtain a part or all of the exercise data.

11. A portable terminal device according to any one of claim 1 through claim 10, wherein a part or all of said specified exercise data is input from said input unit.

12. A portable terminal device according to any one of claim 1 through claim 11, wherein said portable terminal device comprises a clinical data creating section which creates clinical data based on said basic data.

13. A portable terminal device according to claim 12, wherein said clinical data creating section creates a table showing a causal relation with food intake, and graphs the data corresponding to clinical analysis.

14. A portable terminal device according to claim 11, wherein said clinical data creating section creates an acceptable intake on the day, based on a difference between a limited calorific intake per day and ingested calories on the day.

15. A portable terminal device according to any one of claim 1 through claim 14, wherein said portable terminal device comprises a source material creating section which creates source material having the accumulated basic data or clinical data statistically processed.

16. A lifestyle-related disease patient - medical institution cooperation system having; a portable terminal device group comprising portable terminal devices which can be carried by users, an information processor in a patient data management center which manages data sent by the portable terminal device group, and a medical treatment terminal device group in medical institutions which can use the data in said patient data management center and perform medical services with respect to said user, connected via a network, wherein:
said portable terminal device comprises: an input unit which can input life data including the user's food intake contents; a display unit which displays the data; and a communication unit which sends basic data including said life data and receives medical data;
said information processor comprises: a clinical data creating section which creates clinical data based on said basic data; a clinical database which accumulates said clinical data; and a communication unit which receives the basic data and sends the clinical data; and
said medical treatment terminal device comprises a communication unit which receives said clinical data and sends medical data created based on said clinical data.

17. A lifestyle-related disease patient - medical institution cooperation system according to claim 16, wherein said portable terminal device has a measuring unit which can measure a user's in-vivo substance to obtain biological data, and said basic data includes said biological data and said life data.

18. A lifestyle-related disease patient - medical institution cooperation system according to either one of claim 16 and claim 17, wherein said data storage unit is removably provided with respect to said portable terminal device.

19. A lifestyle-related disease patient - medical institution cooperation system according to any one of claim 16 through claim 18, wherein said biological data includes measurement time point data for when said in-vivo substance is measured by said measuring unit.

20. A lifestyle-related disease patient - medical institution cooperation system according to any one of claim 16 through claim 19, wherein said measuring unit in said portable terminal device is an in-vivo substance measuring apparatus which is detachably and data transferably connected to said portable terminal device main body.

21. A lifestyle-related disease patient - medical institution cooperation system according to any one of claim 16 through claim 20, wherein said display unit and said input unit or communication unit in said portable terminal device uses a display unit and an input unit or a communication unit of a portable telephone which is detachably and data transferably connected to said portable terminal device main body.

22. A lifestyle-related disease patient - medical institution cooperation system according to any one of claim 16 through claim 21, wherein said input unit which inputs said food intake contents, previously sets a food intake pattern according to special conditions and a pattern based on lifestyle habits of breakfast, lunch, and dinner, so as to suit the user's dietary habits, and classifies fixed units for each food into levels such as large quantity, medium quantity, and small quantity, to enable selection thereof.

23. A lifestyle-related disease patient - medical institution cooperation system according to any one of claim 16 through claim 22, wherein said basic data includes exercise data showing a user's exercise quantity.

24. A lifestyle-related disease patient - medical institution cooperation system according to any one of claim 16 through claim 23, comprising an exercise quantity measuring apparatus which is formed integrally with said portable terminal device or separately from said portable terminal device, and measures said exercise quantity of a user carrying said device so as to obtain a part or all of the exercise data.

25. A lifestyle-related disease patient - medical institution cooperation system according to any one of claim 16 through claim 24, wherein a part or all of said exercise data is input from said input unit.

26. A lifestyle-related disease patient - medical institution cooperation system according to claim 25, wherein said information processor sends a necessary part of the clinical data created by said clinical data creating section to said medical treatment terminal device group or said portable terminal device group.

27. A lifestyle-related disease patient - medical institution cooperation system according to either one of claim 25 and claim 26, wherein said clinical data creating section in said information processor creates a table showing a causal relation with food intake, and graphs the data corresponding to clinical analysis.

28. A lifestyle-related disease patient - medical institution cooperation system according to any one of claim 25 through claim 27, wherein said clinical data creating section in said information processor creates an acceptable intake on the day as the clinical data, based on a difference between a limited calorific intake per day and ingested calories on the day, and sends it.

29. A lifestyle-related disease patient - medical institution cooperation system according to any one of claim 25 through claim 28, wherein said information processor holds the personal data of the user which has been registered in said information processor based on at least a joining contract with the present system.
